# EUROPEAN PATENT SPECIFICATION

(11) **EP 1 538 445 B1**
(45) Date of publication and mention of the grant of the patent: **17.01.2007**
(21) Application number: 03787825.3
(22) Date of filing: 14.08.2003
(51) Int. Cl.: G01N 33/558, G01N 33/564

(54) **IMMUNOCHROMATOGRAPHY SYSTEM AND METHOD FOR THE SIMULTANEOUS IDENTIFICATION OF AGA AND T-TG ANTIBODIES AND USE THEREOF FOR THE DIAGNOSIS OF CELIAC DISEASE**
IMMUNOCHROMATOGRAPHIESYSTEM UND VERFAHREN ZUR GLEICHZEITIGEN IDENTIFIKATION VON AGA-UND T-TG-ANTIKÖRPERN UND VERWENDUNG DAFÜR ZUR DIAGNOSE DER ZÖLIAKIEKRANKHEIT
SYSTEME ET PROCEDE IMMUNOCHROMATOGRAPHIQUE POUR L'IDENTIFICATION SIMULTANEE D'ANTICORPS FACE A AGA ET T-TG ET LEUR UTILISATION DANS LE DIAGNOSTIC DE LA MALADIE COELIAQUE

(30) Priority: 16.08.2002 ES 200201939
(43) Date of publication of application: 08.06.2005
(73) Proprietor: CONSEJO SUPERIOR DE INVESTIGACIONES CIENTIFICAS, 28006 Madrid (ES); Operon, S.A., 50410 Cuarte de Huerva (ES)
(72) Inventor: MENDEZ CORMAN, E., Centro Nac. Biotecnologia, E-28049 Madrid (ES); GENZOR ASIN, Carlos Gustavo, E-50410 Cuarte de Huerva (ES); GAMEN SIERRA, Susana, E-50410 Cuarte de Huerva (ES); CORBATON PAMPLONA, Vicente Manuel, E-50410 Cuarte de Huerva (ES); NAVARRO GALINDO, Antonio, E-50410 Cuarte de Huerva (ES); VILLACAMPA RUIZ, Manuel, E-50410 Cuarte de Huerva (ES)
(74) Representative: Arias Sanz, Juan
(86) International application number: PCT/ES2003/000425
(87) International publication number: WO 2004/016065

(56) References cited:
- EP-A2- 1 164 375
- ES-A- 2 141 679
- BAZZIGALUPPI E. ET AL: 'Comparison of tissue trans-glutaminase-specific antibody measurements for coelias disease' JOURNAL OF AUTOIMMUNITY vol. 12, no. 1, February 1999, pages 51 - 56, XP001097614
- SORELL L. ET AL: 'One-step immunochromatographic assay for screening or coeliac disease' LANCET vol. 359, no. 9310, March 2002, pages 945 - 946, XP001097616
- PALACIOS S. ET AL: 'Anticuerpo antitransglutaminasa: utilidad en el diagnostico de la engermedad celiaca' AN ESP. PEDIATR. vol. 53, no. 6, July 2000, pages 542 - 546, XP002976803

## Description

### FIELD OF THE INVENTION

The present invention is encompassed in the field of biotechnology, and specifically in the diagnosis of human diseases. The object of this invention is a fast and easy identification of anti-antigen antibodies associated with celiac disease (CD) in human serum, plasma or blood samples.

### STATE OF THE ART

Celiac disease (CD) is an enteropathy characterized by an intolerance to wheat, rye, barley and oatmeal proteins (gluten) in genetically predisposed individuals. The intestinal lesion is immunologically mediated, gliadin being the trigger of a series of cascades causing an activation of the immune system and leading to the production of the flattening of the intestinal mucosa and crypt hyperplasia (Marsh. Correlative Aspects of Mucosal Pathology Across the Spectrum of Gluten-sensitivity. In C-F.A. C. O Farrelly de. Gastrointestinal Immunology and Gluten-sensitive Disease. Dublin: Oak Tree Press, 1994: 145-157). The prevalence of CD, including the symptomatic form as well as the silent or atypical form, has recently been estimated to be 1/160 when the analysis is carried out in the general population. Mortality linked to the higher risk of neoplasias as well as morbidity -including miscarriages, osteopenia, osteoporosis, autoimmune diseases, etc.- associated with untreated CD, requires early diagnosis, the benefits of which have been demonstrated after applying a gluten-free diet. To confirm the enteropathy, a biopsy of the small intestine with a histopathological study, as well as different serological tests for cellular antigens or of foods, which have earned acceptance in the last few years for screening candidates for carrying out said intestinal biopsy, are necessary.

Since tissue transglutaminase (t-TG) was recently identified as the greatest autoantigen present in structures of the endomysium (Dieterich W, Ehnis T, Bauer M et al., Identification of Tissue Transglutaminase as the Autoantigen of Celiac Disease. Nat Med 197; 3: 797-801; WO 98/03872 Immunological Process for Detecting Antibodies Directed Towards Tissue Transglutaminase (TTG), Use of TTG for Diagnostic Purposes and Therapy Control, and Oral Pharmaceutical Agent Containing TTG, Schuppan D, Dieterich W), the t-TG ELISA technique is the most universally accepted technique for testing in the diagnosis of celiac disease (CD) in diagnosis. Other authors propose an immunological assay in which the antibodies of CD patients would react better with an antigen formed by t-TG and a substrate thereof (WO 01/29090, IMMCO DIAGNOSTICS, Immunological Assay for Detection of Antibodies in Celiac Disease).

On the other hand, a one-step immunochromatographic assay has been disclosed for the detection of IgA and IgG type antibodies to t-TG in human plasma or serum (Sorell L, Garrote JA, Acevedo B, et al., One-step Immunochromatographic Assay for Screening of Coeliac Disease. Lancet 2002; 359: 945-946). According to these authors, the anti t-TG immunochromatographic assay had a 100% sensitivity and specificity in the diagnosis of CD, i.e. data similar to that obtained with intestinal biopsy constituting the gold standard test in the diagnosis of CD. However, it must be pointed out that said work was carried out with a small group of patients with CD (n= 50), as a result of which these results must be considered as being excessively promising. These same authors filed a patent related to the theoretical use of t-TG in the elaboration of an immunochromatographic assay for the diagnosis of CD, although they did not indicate any practical embodiment of said assay nor any specific results with samples from patients with CD (EP 1,164,375, Assay for Anti-transglutaminase Antibodies, Sorell LT, Aroya H, Acevedo BE, Cerro H).

Antigliadin antibodies (AGA) are also indicative of CD, although the specificity of the anti-AGA ELISA assays carried out is less than that with other assays, and separate assays must be carried out for the identification of the IgA and IgG antibodies. Recently, there has also been an AGA visual diagnosis assay (Garrote JA, Sorell L, Alfonso P et al., 1999. A Simple Visual Immunoassay for the Screening of Coeliac Disease. Eur. J Clin Invest 29; 697-699) and a solid phase immunoassay for antibodies specific for AGA (ES 2141679). Another patent related to the diagnosis of CD by means of the identification of antigliadin antibodies is patent WO 00/25793 (Diagnosis of Coeliac Disease Using a Gliadin Epitope, Anderson R, Hill A, Jewell D), although it is a laborious method as T lymphocytes are handled *in vitro*.

Bazzigaluppi et al. (Bazzigaluppi E. et al., Journal of Autoimmunity, Vol. 12, No. 1, Feb. 1999, pp 51-56) disclose a radiobinding assay for IgG and IgA specific for t-TG; IgA and IgG for AGA is measured for competitive reasons with immunoenzymatic methods. ELISA methods for measuring IgA for AGA and IgA for t-TG have been also described (Palacios E. et al., An. Esp. Pediatr. vol. 53, No. 6, July 2000, pp 542-546).

Due to the foregoing, even though the sensitivity and specificity of the serological markers, such as anti-gliadin antibodies (AGA), anti-endomysium antibodies (EmA) and anti-tissue transglutaminase (t-TG) antibodies can be high, carrying out said ELISA and immunofluorescence techniques require well-equipped laboratories and qualified staff, which may not be available worldwide, as well as the repetition of the techniques for a single biological sample to identify the different IgA, IgG or IgM.

Therefore, the interest arises from this to develop a new, more economical and easier to perform assay method to be used in the diagnosis of CD, especially in populations at risk. Until now, only a few of these assays have been developed, the majority of which are based on the serological response to gliadin and, finally, to t-TG, which have been determined to be the most frequent antibodies associated to CD. The process disclosed in this patent is the first visual process using recombinant human t-TG, and the first visual process which allows detecting IgA type antibodies to t-TG and AGA in a single assay, i.e. with a single immunochromatographic strip.

### DESCRIPTION

### Brief Description

An object of the present invention is constituted of a double immunochromatography system allowing the implementation of the previously disclosed process and simultaneously including the necessary elements for determining and visualizing the immunochromatographic (IC) reactions between IgA antibodies (double AGA/t-TG strip) and IgG antibodies or IgM antibodies (anti t-TG IgA/IgG/IgM strip) that are characteristic of celiac patients with at least two CD immune response inductive antigens.

Another object of the present invention is a process for the identification of antibodies present in patients with celiac disease by means of an immunochromatography system jointly and simultaneously assessing the presence in a biological sample of antibodies - IgA, IgG or IgM, in each system separately- specific to the autoantigens that are characteristic of CD, AGA and t-TG.

Finally, an object of the present invention is constituted of the use of the immunochromatography system and process of the present invention for the identification of antibodies present in patients with celiac disease.

### Detailed Description

The present invention is based on the fact that the inventors have observed that the joint and simultaneous identification of antibodies present in patients with celiac disease is possible by means of an immunochromatography system (immunochromatographic strips) with better sensitivity and specificity values than those carried out separately, and similar to those obtained by other immunological techniques, for example ELISA. Thus, the presence of antibodies to different autoantigens of CD, specifically AGA and t-TG, has been able to be determined in a single immunochromatographic strip and with the same reaction conditions; and for those cases in which the CD patients have an absence of IgA, another immunochromatographic strip has been developed based on the same t-TG autoantigen of the previous strip.

An object of the present invention is constituted of a double immunochromatography system allowing the implementation of the previously described process and simultaneously including the necessary elements for the determination and visualization of the immunochromatographic (IC) reactions between antibodies that are characteristic of celiac patients with at least two CD immune response inductive antigens. Specifically, said immunochromatography system for the diagnosis of celiac disease in humans is formed by the following elements:
I) an immunochromatographic strip allowing the identification of antibodies -IgA type- specific to the autoantigens that are characteristic of CD, AGA and t-TG, in a human biological sample, comprising
   a) a system for visualizing the reaction, for example, colloidal particles or colored microspheres coated with an anti IgA monoclonal antibody,
   b) AGA and t-TG antigens immobilized on the same strip, and
   c) a control system for the immunochromatographic reaction conditions,
   and,
II) an immunochromatographic strip allowing the identification of IgA/IgM/IgG antibodies to the autoantigen, t-TG, in a human biological sample, comprising
   a) a system for visualizing the reaction, for example, colloidal particles or colored microspheres coated with t-TG antigen,
   b) t-TG antigen immobilized on the same strip, and
   c) a control system for the immunochromatographic reaction conditions.

As it is used in the present invention, the term "biological sample" refers to a serum, plasma, saliva or blood type biological sample from a patient suspected of having CD.

As it is used in the present invention, the term "AGA" refers to a protein extract obtained from one or from a mixture of several cereals belonging to the following group - wheat, barley, rye and oat- and which induces antibodies in patients with CD.

As it is used in the present invention, the term "t-TG" refers to transglutaminase protein of animal or human origin, synthetic or recombinant, and also to fragments of said t-TG which induce an immunological response in patients with CD which is similar to that obtained with complete t-TG.

A particular object of the present invention is an immunochromatography system in which the AGA antigen has been obtained from a cereal variety or mixture of cereals, such as for example the Triana wheat variety and the t-TG antigen is a recombinant human t-TG (see Example 1).

Thus, a particular embodiment of the present invention is constituted of a double lateral flow immunochromatographic strip as an inert support determining and displaying the IC reactions between IgA and AGA and IgA and t-TG on a single strip carried out according to Example 1 a) and comprising a system for visualizing the reaction (for example, colloidal particles or colored microspheres coated with an anti human IgA monoclonal antibody), the immobilization of the CD antigens, AGA and t-TG (in this case, t-TG), an inert support allowing the flow of said elements which are reconstituted when the serum or plasma is added, and a control system for the immunochromatographic reaction conditions. On the other hand, any other double IC strip which can be developed from the current state of the art in the field of immunochromatography techniques and known by persons skilled in the art, forms part of the invention.

Another further particular embodiment of the present invention is constituted of an immunochromatographic strip (anti t-TG IgA/IgG/IgM strip, see example 1 b)) allowing the determination of IgA or IgG or IgM antibodies of patients with CD against the t-TG autoantigen used on the previously mentioned double AGA/t-TG strip.

Another object of the present invention is a process for the identification of antibodies present in patients with celiac disease by means of an immunochromatography system assessing in a combined and simultaneous manner the presence of antibodies -IgA, as well as IgM or IgG, separately on each immunochromatographic strip- which are specific to the autoantigens that are characteristic of CD, AGA and t-TG, in a biological sample by means of two different immunochromatographic strips (double AGA/t-TG strip and anti t-TG IgA/IgG/IgM strip).

A particular object of the present invention is constituted of a previously described process in which rather than simultaneously carrying out the assessment of CD-specific antibodies by means of the two immunochromatographic strips, the analysis of the antibodies is first carried out by means of the double AGA/t-TG immunochromatographic strip (Example 1 a)), and in the event of a negative result, a second assessment of the presence of IgA, IgG and IgM type antibodies to t-TG is carried out by means of a single immunochromatography system (Example 1 b)).

Another particular object of the present invention is constituted of a process according to this patent in which the immunochromatographic reaction carried out occurs between the CD autoantigens arranged on the immunochromatographic strip, AGA and t-TG, and IgG or IgM antibodies of the biological sample separately. In these cases, the colloidal particles or colored microspheres of the double strip would be coated with a monoclonal antibody recognizing human IgG or IgM, respectively, for each one of the strips.

Finally, an object of the present invention is constituted of the use of the immunochromatography system constituted of the immunochromatographic strips and of the process of the present invention for the identification of antibodies (IgA, IgM or IgG) present in patients with celiac disease.

### DESCRIPTION OF THE DRAWINGS

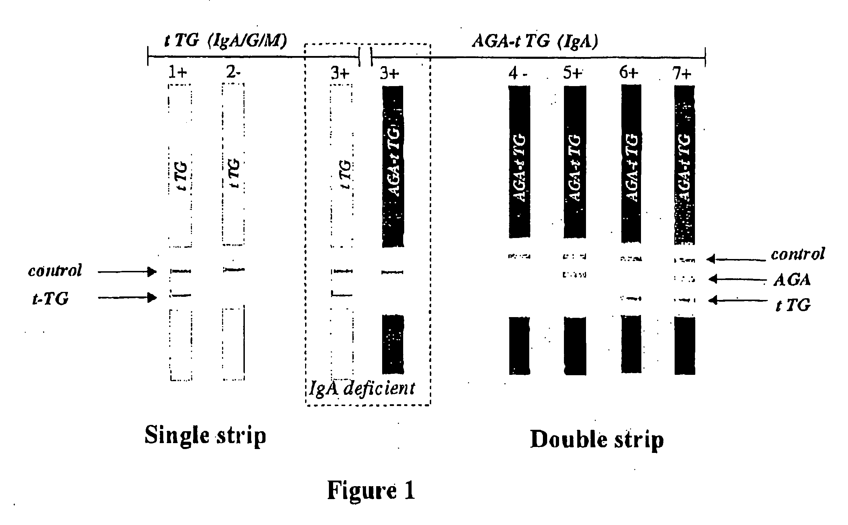

**Figure 1.- Results of the double and single immunochromatographic strips in the diagnosis of CD.** This figure shows the representation of the bands displayed after the immunochromatographic assays with the double strip (AGA/t-TG) and single strip (IgA/IgG/IgM). 1+, t-TG positive strip by means of single strip; 2+, t-TG negative strip by means of single strip; 3+ and 3-, comparison of a single and double strip in a patient with IgA deficiency; 4-, t-TG/AGA negative strip on a double strip; 5+, AGA positive strip on a double strip; 6+, t-TG positive strip on a double strip; 7+, AGA/t-TG strip positive on a double strip.

### EMBODIMENT EXAMPLES

### Example 1.- Elaboration of the immunochromatographic strips

The process developed in the present invention is based on lateral flow immunochromatographic strips produced by the company Operon, S.A. (Zaragoza, Spain). The two strip models elaborated are presented below: a) AGA and t-TG IgA type strips, and b) t-TG IgA/IgG/IgM strips. The strips consist of several layers provided with all the necessary reagents in each type.

### a) Double AGA/t-TG strip

This is a three band test (see Figure 1). The human serum samples, from CD patients or from control cases, are diluted in a buffering solution containing bovine serum albumin (BSA) and a non-ionic detergent, being possible to carry out said dilution either in Eppendorf tubes or on microtiter plates. The absorbent region of the strip or stick is introduced in the 1/15 dilution of the previously mentioned buffered serum. If the serum of the patient has IgA type antibodies to t-TG, gliadins or both, when the liquid moves upwards due to capillarity (lateral flow immunochromatographic strip), it reconstitutes colored microspheres located in this absorbent region of the strip, and which in this case are coated with a monoclonal antibody to human IgA. Once reconstituted, the complex formed by the red microspheres coated with a monoclonal antibody to human IgA, the antibodies (IgA type) to t-TG, or to gliadins or to both antigens, moves upwards and reacts separately with two different lines (physically separated), containing both immobilized purified gliadins (in one of the lines) and recombinant human t-TG (in the other line), which results in one or two red bands, after the corresponding immunological reaction, at different levels, depending on whether the serum has antibodies (IgA type) to t-TG, to gliadins (AGA) or to both antigens. In the event of immunoreactivity, apart from the blue-colored control band in this example, a red band can develop in this example after 5-10 minutes which corresponds to t-TG, or to AGA, or two bands corresponding to IgA type antibodies to AGA and t-TG can develop. The blue-colored control band appears at the top portion of the strip validating the result of the test, the red band corresponding to the presence of IgA type antibodies to gliadin (AGA) in the middle portion, and the red band indicating the presence of IgA type antibodies to t-TG appears in the lower portion of the strip.

The blue-colored band is an internal control of the immunochromatographic strip indicating that the conditions necessary for the antigen-antibody reaction and the subsequent visualization thereof are suitable.

The recombinant human t-TG (Rt-TG) was obtained from Diarect (Freiburg, Germany), and the purified gliadin was isolated from a wheat variety, the Triana variety, by the inventors themselves at the Unidad de Gluten del Centro Nacional de Biotechnologia. This purification was carried out by means of an ethanolic extraction, followed by a concentration using ultrafiltration, and subsequently lyophilizing the extract. The monoclonal antibody to human IgA was produced at Operon, S.A. (Zaragoza, Spain).

With this type of strips, 248 serums were tested, 50% of them corresponding to patients with CD and the other 50% corresponding to non-CD control cases, in the same manner as the single strips.

### b) Single anti t-TG IgA/IgG/IgM strips

This is a two-band test. The sticks or strips are introduced in Eppendorf tubes or in microtiter plates containing a previously prepared 1:20 dilution of serum from patients to be studied with a saline phosphate buffer solution (PBS) at a physiological saline concentration and pH with 1% bovine serum albumin (BSA). When the strip is placed in the diluted sample, the liquid moves upwardly due to capillarity and reconstitutes the red-colored microspheres which are dried and coated with recombinant human t-TG (Rt-TG) if the sample contains antibodies to tissue transglutaminase (t-TG). The complex formed by the red-colored microspheres, the recombinant human t-TG and the anti-t-TG antibodies present in the patient sample, moves upwards and reaches a region containing a recombinant human t-TG line, causing an immunoreaction resulting in a red band. This red band indicates the presence of anti t-TG IgA, IgG or IgM type antibodies in the analyzed sample. Other dried, blue-colored microspheres coated with a certain protein are located in the absorbent region of the stick as a system control. The reconstituted protein coated blue-colored microsphere complex moves upwardly on the stick until reaching a region in which there are immobilized monoclonal antibodies to said protein, immunologically reacting and resulting in a blue band. When the serum is not immunoreactive, only a blue control band develops, whereas in the event of immunoreactivity, in addition to the blue band, a red band develops after 5-10 minutes.

In the event that neither of the two bands appears, the analysis is not valid and it must be repeated using a new strip. No additional equipment is necessary for carrying out the assay.

### Example 2.- Immunochromatographic and ELISA assays for CD

The results obtained in the tests with the two types of strips, double and single, were compared with the results obtained by ELISA for anti t-TG and AGA antibodies.

In the case of the double diagnosis strip, a 92.7% and 83.2% sensitivity, with a 96.2% and 96.7% specificity for t-TG and AGA were found, respectively, when the t-TG and AGA results were analyzed separately (Table 1). This data showed a consistency of 99.3% and 99.2%, respectively, compared to ELISA. On the other hand, it is necessary to point out that the combined analysis of the results on the same double strip, i.e. number of cases that screened positive to one of the two antigens, AGA and t-TG, separately, or both resulting in positive, allowed for obtaining higher sensitivity and specificity values than those observed separately, 95.2% and 96.2%, respectively. Specifically, it was observed that three patients with CD with positive AGA on the double strip were t-TG negative, fifteen patients with CD with positive t-TG were AGA negative, and one hundred patients with CD were positive for AGA and t-TG. In other words, the embodiment of a double strip allowed for identifying three patients with CD who would have been possible to identify with a separate AGA or t-TG analysis (this is what increases the sensitivity from 92.7% to 96.3%).

In the negative CD cases to AGA/t-TG obtained with the double strip, the presence of IgA/IgM/IgG antibodies to t-TG was analyzed by carrying out an immunochromatographic assay with the single strip, one positive case to t-TG being observed in which the IgA deficiency was confirmed in this patient by means of the nephelometry technique. One of the most remarkable advantages of the combined use of both double and single strips is that it allows for the detection of patients with selective IgA deficiency, and who have CD, when the t-TG band is positive in the single strip and negative in the double strip, whereby the sensitivity obtained by both tests increases (in our case, from 95.2% to 96%).

**Table 1**

| **Double AGA/t-TG** | **Sensitivity** | **Specificity** | **Consistency** | **Serum** |
|---|---|---|---|---|
| **strip (IgA)** | **(%)** | **(%)** | **(%)** | **(n)** |
| **Separate interpretation of AGA and t-TG** | | | | |
| | | | | |
| ***t-TG strip* (IgA)** | 92.7 | 96.2 | 99.3 | 248 |
| ***t-TG (ELISA)* (IgA)** | 93.6 | 95.27 | | |
| ***AGA strip* (IgA)** | 83.2 | 96.7 | 99.2 | 248 |
| ***AGA ELISA* (IgA)** | 84.8 | 92.8 | | |

| **Combined interpretation of AGA and t-TG** | | | | |
|---|---|---|---|---|
| **Double* AGA/t-TG** | 95.2 | 96.7 | | 248 |
| **strip (IgA)** | | | 99.2 | |
| **TG (IgA)** | | | | |
| **AGA ELISA(IgA)** | 96.0 | 96.7 | | 248 |
| **+t-TG ELISA (IgA)*** | | | | |

| | | | | |
|---|---|---|---|---|
| • A single double strip for two ELISA tests. | | | | |

As can be seen in the table, there is a high correlation between the different visual assays and the respective ELISA tests.

The results obtained in the immunochromatographic assays presented show these methods to be extremely efficient for the diagnosis of CD, which, combined with the easiness, the fact that no specially qualified operator is required, the fact that it is quick to carry out (no more than 10 minutes), and the fact that it does not require complex technological equipment, make the method a very useful tool for inspecting populations at risk for CD and for screening candidates for carrying out a small intestine biopsy.

Another important feature is that these strips have higher sensitivity and specificity values than the corresponding t-TG and AGA ELISA tests, being extremely efficient for the testing and diagnosis of CD.

The double AGA/t-TG strip determines IgA type AGA and t-TG in a single analysis, therefore eliminating the need to use two parallel t-TG and AGA ELISA tests.

Both strips are significantly less expensive than ELISA, simpler to handle and interpret, and it is believed that these features can make it an ideal test for the diagnosis of CD in large populations, especially in developing countries.

## Claims

1. An immunochromatography system for the diagnosis of celiac disease (CD) in humans, which comprises the following elements:
I) an immunochromatographic strip allowing the identification of antibodies -IgA type- specific to the autoantigens that are characteristic of CD, AGA (antigliadin) and t-TG (tissue transglutaminase), in a human biological sample, comprising
a) a system for visualizing the reaction comprising an anti-IgA antibody, for example, colloidal particles or colored microspheres coated with an anti-IgA monoclonal antibody,
b) AGA and t-TG antigens immobilized on the same strip, and
c) a control system for the immunochromatographic reaction conditions,
and,
II) an immunochromatographic strip allowing the identification of IgA/IgM/IgG antibodies to the autoantigen t-TG, in a human biological sample, comprising
a) a system for visualizing the reaction comprising t-TG antigen, for example, colloidal particles or colored microspheres coated with t-TG antigen,
b) t-TG antigen immobilized on the same strip, and
c) a control system for the immunochromatographic reaction conditions.

2. - An immunochromatography system according to claim 1, wherein the t-TG is a recombinant human t-TG and the AGA is a protein extract obtained from a wheat variety.

3. - An immunochromatographic process for identifying antibodies present in patients with celiac disease (CD), which comprises assessing:
a) the simultaneous presence of antibodies -IgA type-specific to the autoantigens that are characteristic of CD, AGA (antigliadin) and t-TG (tissue transglutaminase), in a biological sample by means of an immunochromatographic strip according to claim 1 I),
and
h) the presence of IgA/IgM/IgG antibodies to the autoantigen t-TG, in the same biological sample as in a) by means of an immunochromatographic strip according to claim 1 II).

4. - A process according to claim 3, wherein the biological sample consists of serum, plasma, saliva or blood from a patient suspected of having CD.

5. - An immunochromatographic process for identifying antibodies present in patients with celiac disease (CD), which comprises:
a) assessing the presence of antibodies -IgA type- specific to the autoantigens that are characteristic of CD, AGA (antigliadin) and t-TG (tissue transglutaminase), in a biological sample by means of an immunochromatographic strip according to claim 1 I), and, in the event of a negative result,
b) assessing the presence of IgA, IgM and IgG antibodies to the autoantigen t-TG, in the same biological sample as in a) by means of an immunochromatographic strip according to claim 1 II) .

6. - Use of the immunochromatographic system according to claims 1 and 2 and of the process according to claims 3 to 5 for the identification of antibodies (IgA, IgM or IgG) present in samples taken from patients with celiac disease.

## Patentansprüche

1. Immunchromatographisches System für die Diagnose der Zöliakie (CD) beim Menschen, das die folgenden Elemente umfaßt:
I) einen immunchromatographischen Streifen, der die Identifizierung von Antikörpern - vom IgA-Typ - spezifisch für die Autoantigene, die für CD charakteristisch sind, AGA (Antigliadin) und t-TG (Gewebstransglutaminase) in einer menschlichen biologischen Probe ermöglicht, umfassend:
a) ein System zum Visualisieren der Reaktion, umfassend einen anti-IgA-Antikörper, beispielsweise kolloidale Teilchen oder gefärbte Mikrosphären, beschichtet mit einem anti-IqA monoklonalen Antikörper,
b) AGA und t-TG-Antigene, immobilisiert auf demselben Streifen und
c) ein Kontrollsystem für die immunchromatographischen Reaktionsbedingungen,
und
II) einen immunchromatographischen Streifen, der die Identifizierung von IgA/IgM/IgG-Antikörpern gegen das Autoantigen t-TG in einer menschlichen biologischen Probe ermöglicht, umfassend:
a) ein System zum Visualisieren der Reaktion, umfassend das t-TG-Antigen, beispielsweise kolloidale Teilchen oder gefärbte Mikrosphären, beschichtet mit t-TG-Antiqen,
b) t-TG-Antigen, immobilisiert auf demselben Streifen und
c) ein Kontrollsystem für die immunchromatographischen Reaktionsbedingungen,

2. Immunchromatographisches System gemäß Anspruch 1, wobei das t-TG ein rekombinantes menschliches t-TG und das AGA ein Proteinextrakt, erhalten von einer Weizenvarietät, ist.

3. Intrnunctlromatographisches Verfahren zur Identifikation von Antikörpern, die bei Patienten mit Zöliakie (CD) vorliegen, das die Bewertung umfaßt :
a) der simultanen Gegenwart von Antikörpern - vom IgA-Typ - spezifisch für die Autoantigene, die für CD charakteristisch sind, AGA (Antigliadin) und L-TG (Gewebstransglutaminase), in einer biologischen Probe durch ein immunchromatographischen Streifen gemäß Anspruch 1 I), und
b) der Gegenwart von TgA/IgM/IgG-Antihörpern gegen das Autoantigen t-TC in derselben biologischen Probe wie bei a) durch einen immunchromatographischen Streifen gemäß Anspruch 1 II).

4. Verfahren gemäß Anspruch 3, wobei die biologische Probe aus Serum, Plasma, Speichel oder Blut von einem Patienten stammt, von dem angenommen wird, daß er CD hat.

5. Immunchromatographisches Verfahren zur Identifizierung von Antikörpern, die bei Patienten mit Zöliakie (CD) vorliegen, das folgendes umfaßt:
a) Bewertung der Gegenwart von Antikörpern - vom IgA-Typ - spezifisch für die Autoantigene, die für CD charakteristisch sind, AGA (Antigliadin) und t-TG (Gewebstransglutaminase) in einer biologischen Probe durch einen immunchromatographischen Streifen gemäß Anspruch 1 1), und im Fall eines negativen Ergebnisses
b) Bewertung der Gegenwart von IgA-, IgM- und IgG-Antikörpern gegen das Autoantigen t-TG in derselben biologischen Probe wie bei a) durch einen immunchromatographischen Streifen gemäß Anspruch 1 II).

6. Verwendung des immunchromatographischen Systems gemäß den Ansprüchen 1 und 2 und des Verfahrens gemäß den Ansprüchen 3 bis 5 für die Identifizierung von Antikörpern (IgA, IgM oder IgG), die in Proben vorliegen, die von Patienten mit Zöliakie entnommen wurden.

## Revendications

1. Système immunochromatographique pour le diagnostique de la maladie coeliaque (MC) chez des humains, qui comprend les éléments suivants :
I) une bande immunochromatographique permettant l'identification d'anticorps de type IgA spécifiques aux auto-antigènes qui sont caractéristiques de MC, AGA (antigliadine) et t-TG (transglutaminase de tissu), dans un échantillon biologique humain, comprenant
a) un système de visualisation de la réaction comprenant un anticorps anti-IgA, par exemple, des particules colloïdales ou des microsphères colorées enrobées d'un anticorps monoclonal anti-IgA,
b) des antigènes AGA et t-TG immobilisés sur la même bande, et
c) un système de contrôle pour les conditions de réaction immunochromatographique, et,
II) une bande immunochromatographique permettant l'identification d'anticorps IgA/IgM/IgG sur l'auto-antigène t-TG, dans un échantillon biologique humain, comprenant
a) un système de visualisation de la réaction comprenant un antigène t-TG, par exemple, des particules colloïdales ou des microsphères colorées enrobées avec l'antigène t-TG,
b) l'antigène t-TG immobilisé sur la même bande, et
c) un système de contrôle pour les conditions de réaction immunochromatographique.

2. Système immunochromatographique selon la revendication 1, dans lequel la t-TG est une t-TG humaine recombinante et l'AGA est un extrait de protéine obtenu d'une variété de blé.

3. Processus immunochromatographique pour l'identification d'anticorps présents chez des patients atteints de la maladie coeliaque (MC), qui comprend l'étape consistant à évaluer :
a) la présence simultanée d'anticorps de type IgA spécifiques aux auto-antigènes qui sont caractéristiques de MC, AGA (antigliadine) et t-TG (transglutaminase de tissu), dans un échantillon biologique au moyen d'une bande immunochromatographique selon la revendication 1 I),
et
b) la présence d'anticorps IgA/TgM/TgG sur l'auto-antigène t-TG, dans le même échantillon biologique que dans a) au moyen d'une bande immunochromatographique selon la revendication 1 II).

4. Processus selon la revendication 3, dans lequel l'échantillon biologique consiste en du sérum, du plasma, de la salive ou du sang d'un patient suspecté d'être atteint de MC.

5. Processus immunochromatographique pour l'identification d'anticorps présents chez des patients atteints de la maladie coeliague (MC), qui comprend:
a) évaluer la présence d'anticorps de type IgA spécifiques aux auto-antigènes qui sont caractéristiques de MC, AGA (antigliadine) et t-TC (transglutaminase de tissu), dans un échantillon biologique au moyen d'une bande immunochromatographique selon la revendication 1 I), et, dans le cas d'un résultat négatif,
b) évaluer la présence d'anticorps IgA, IgM et IgG sur l'auto-antigène t-TG, dans le même échantillon biologique que dans a) au moyen d'une bande immunochromatographique selon la revendication 1 II).

6. Utilisation du système immunochromatographique selon les revendications 1 et 2 et du processus selon les revendications 3 à 5 pour l'identification des anticorps (IgA, IgM ou IgG) présents dans des échantillons prélevés sur des patients atteints de la maladie coeliaque.
